# EUROPÄISCHE PATENTSCHRIFT

(11) **EP 0 646 026 B1**
(45) Veröffentlichungstag und Bekanntmachung des Hinweises auf die Patenterteilung: **15.01.1997**
(21) Anmeldenummer: 93912700.7
(22) Anmeldetag: 07.05.1993
(51) Int. Cl.: A61M 5/32

(54) **SCHUTZHÜLLE ZUR ENTSORGUNG EINER GEBRAUCHTEN EINMALKANÜLE**
PROTECTIVE SHEATH FOR THE DISPOSAL OF USED DISPOSABLE HOLLOW NEEDLES
HOUSSE DE PROTECTION POUR L'ELIMINATION APRES UTILISATION D'UNE CANULE A AIGUILLE A USAGE UNIQUE

(30) Priorität: 07.05.1992 DE 4214493
(43) Veröffentlichungstag der Anmeldung: 05.04.1995
(73) Patentinhaber: HIEKE, Kurt, D-85540 Haar (DE); GRUNDKE, Reinhold, D-84489 Burghausen (DE); Arent, Gerhard, D-81735 München (DE)
(72) Erfinder: HIEKE, Kurt, D-85540 Haar (DE); GRUNDKE, Reinhold, D-84489 Burghausen (DE); Arent, Gerhard, D-81735 München (DE)
(74) Vertreter: Hieke, Kurt
(86) Internationale Anmeldenummer: EP9301121
(87) Internationale Veröffentlichungsnummer: WO9321979

(56) Entgegenhaltungen:
- EP-A- 0 381 577
- WO-A-91/07199
- WO-A-91/08786
- DE-A- 4 013 013
- DE-U- 8 703 046
- FR-A- 2 649 893
- US-A- 4 950 242

## Beschreibung

Die Erfindung bezieht sich auf eine Schutzhülle gemäß dem Oberbegriff des Patentanspruchs 1.

Schutzhüllen dieser Art sind bekannt ( DE-GM 87 03 046, US 49 50 242, EP 03 81 577 A2, WO 91 08 786 und FR 26 49 839).

Die bekannten Schutzhüllen beseitigen die Gefahr, daß sich die die Kanüle im Anschluß an eine Injektion entsorgende Person beim Entsorgen an der Nadelspitze verletzt und dadurch infiziert. Bei den bekannten Schutzhüllen besteht aber noch die Gefahr, daß beim Durchdrücken der Nadel durch den seitlichen Durchgang Flüssigkeit, die nach dem Gebrauch der Kanüle an der Nadel, insbesondere an der Nadelspitze, hängt, in den Raum außerhalb der Schutzhülle versprüht wird, wenn der Verschluß und/oder die Nadel zurückfedern. Die Flüssigkeitströpfchen, die hochinfektiös sein können, verschmutzen die Umgebung und gefährden sowohl die die Entsorgung vornehmende Person als auch andere Personen, die mit ihnen in dem verschmutzten Bereich in Berührung kommen.

Der Erfindung liegt die Aufgabe zugrunde, die bekannten Schutzhüllen gemäß dem einleitenden Teil des Patentanspruchs 1 dahingehend zu verbessern, daß auch die Gefahr des Verspritzens von Flüssigkeit beim Entsorgen einer gebrauchten Kanüle beseitigt ist.

Die vorstehende Aufgabe wird durch die im Kennzeichnungsteil des Patentanspruchs 1 genannten Merkmale gelöst.

Bei der erfindungsgemäßen Schutzhülle ist die die Kanüle entsorgende Person gezwungen, die Nadelspitze in den von der Schutzwand umschlossenen Einführraum oberhalb des Endbereiches des seitlichen Durchgangs einzubringen, bevor sie die Kanüle mit der Nadel durch den Durchgang hindurch in den Aufnahmeraum in der Schutzhülle drücken kann. Die Eintrittsöffnung zu diesem Einführraum kann problemlos so groß gemacht werden, daß die entsorgende Person mit der Spitze der Nadel der noch am Spritzenhals steckenden Kanüle leicht hineinfindet. Die Eintrittsöffnung befindet sich auch in sicherer Entfernung von den die Schutzhülle haltenden Fingern der entsorgenden Person, so daß für diese keine Gefahr besteht, daß sie dabei mit der Nadel in Berührung kommt oder sich gar sticht. Wegen der erfindungsgemäßen Bemessung des Abstandes zwischen der Eintrittsöffnung zum Einführraum und dem Nadelabstreifer muß die Nadelspitze erst in diesen Raum eingebracht worden sein, bevor der Kanülenkopf schutzhülleneinwärts vor den Kanülenabstreifer bewegt werden kann, was zu geschehen hat, ehe die Nadel durch den seitlichen Durchgang hindurch in den Aufnahmeraum gedrückt wird. Die Nadelspitze befindet sich somit vor dem Durchdrücken der Kanüle in den Aufnahmeraum zwangsläufig in dem von der Spritzschutzwandung umgebenen Einführraum, so daß mit Sicherheit ein Verspritzen von möglicherweise an der Nadelspitze hängender Flüssigkeit in die Umgebung ausgeschlossen ist. Die Erfindung schafft damit ein voll taugliches und auch sehr kostengünstiges Entsorgungsmittel für gebrauchte Kanülen, das nur einen geringen Platzbedarf hat und schon der sterilen Einmalkanüle vor deren Benutzung beigegeben werden kann, um dann der eine Injektion verabreichenden oder eine Blutentnahme vornehmenden Person ohne deren Zutun an jedem Einsatzort unmittelbar zur Verfügung zu stehen. Von der in die Schutzhülle entsorgten Kanüle geht keine Infektionsgefahr mehr aus. Die geschützten gebrauchten Kanülen können gefahrlos gesammelt und der weiteren Entsorgung zugeführt werden.

Die Unteransprüche betreffen bevorzugte Ausführungsformen der Schutzhülle gemäß Patentanspruch 1.

Die Erfindung wird nachstehend anhand der Zeichnung an Ausführungsbeispielen noch näher erläutert. In der Zeichnung zeigt:
- Fig.1: eine erste Ausführung der Schutzhülle in Seitenansicht,
- Fig.2: die Schutzhülle nach Fig. 1 von oben gesehen,
- Fig.3: die Schutzhülle gemäß Fig. 1 und Fig. 2 in Stirnansicht für den Betrachter der Fig. 1 von rechts gesehen,
- Fig.4: die Schutzhülle gemäß Fig. 1 - 3 im Schnitt entlang der Schnittlinie IV-IV in Fig. 2,
- Fig.5: eine zweite Ausführung der Schutzhülle in Seitenansicht,
- Fig.6: die Schutzhülle nach Fig. 5 in Ansicht von oben,
- Fig.7: eine besondere Ausführung des Durchgangsverschlusses in Draufsicht,
- Fig.8: eine dritte Ausführung der Schutzhülle in Seitenansicht,
- Fig.9: die Schutzhülle gemäß Fig. 8 in Stirnansicht für den Betrachter der Fig. 8 von rechts gesehen.
- Fig.10: eine weitere Ausführung der Schutzhülle in Seitenansicht,
- Fig.11: die Schutzhülle nach Fig. 10 von oben gesehen,
- Fig. 12: die Schutzhülle gemäß Fig. 10 und Fig. 11 in Stirn ansicht für den Betrachter der Fig. 10 von rechts gesehen,
- Fig.13: die Schutzhülle gemäß Fig. 10 - 12 im Schnitt entlang der Schnittlinie IV-IV in Fig. 11,
- Fig.14: eine weitere Ausführung der Schutzhülle mit nur einem Aufnahmeraum sowohl für die sterile als auch für die gebrauchte Nadel in Seitenansicht, wobei der Gebrauchszustand mit der sterilen Nadel dargestellt ist,
- Fig.15: die Ausführung gemäß Fig. 14 in gleicher Ansicht wie in Fig. 14 mit dem Unterschied, daß hier der Gebrauchszustand mit der entsorgten gebrauchten Nadel dargestellt ist, und
- Fig.16: die Schutzhülle gemäß Fig. 14 und 15 in Stirnansicht für den Betrachter der Fig. 14 und 15 von rechts gesehen.

In der Zeichnung sind gleiche Teile mit gleichen, bzw. mit entsprechenden um den Zahlenwert 100 erhöhten Bezugszeichen bezeichnet.

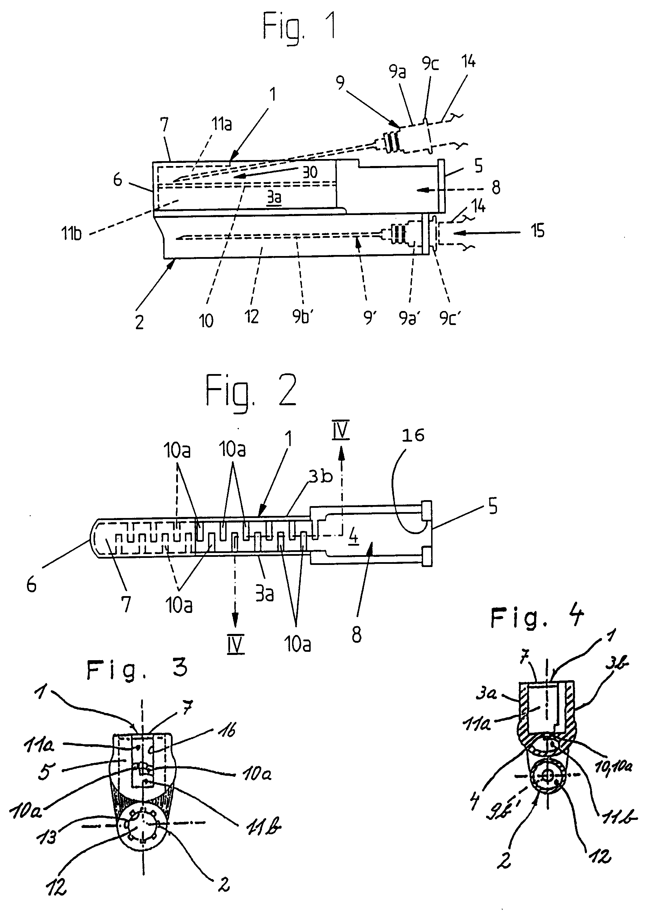

Die Schutzhülle gemäß Fig. 1 - 4 weist einen oberen Teil 1 und einen unteren Teil 2 auf, die aus Kunststoff bestehen und in einem Stück z.B im Spritzgußverfahren hergestellt sein können. Der obere Teil 1 hat Seitenwände 3a, 3b, einen Boden 4, eine vordere Stirnwand 5, eine hintere Stirnwand 6 und eine Deckwand 7, die sich, ausgehend von der hinteren Stirnwand 6 über einen Teil der Hüllenlänge zwischen den Stirnwänden 5 und 6 erstreckt. In einem an die vordere Stirnwand 5 angrenzenden Bereich der Hüllenlänge bilden die Seitenwände 3a, 3b mit dem Boden 4 eine für den Betrachter der Fig. 1 nach oben offene Aufnahmebox 8 für den Kopf 9a einer Kanüle 9, deren Nadel mit 9b bezeichnet ist.

Im Abstand von der Deckwand 7 und vom Boden 4 erstreckt sich zwischen der Aufnahmebox 8 und der hinteren Stirnwand 6 eine Zwischenwand 10, die sich vorzugsweise parallel zum Boden 4 erstreckt und aus reißverschlußartig ineinandergreifenden Zungen 10a gebildet ist, die wechselweise von den Seitenwänden 3a, 3b ausgehen und elastisch zum Boden 4 hin abbiegbar sind. Die Zwischenwand 10 bildet einen elastisch nachgiebigen Verschluß in einem seitlichen Durchgang zu einem Aufnahmeraum 11b für die zu entsorgende gebrauchte Kanüle9. Bei der Ausführung nach Fig. 1 - 4 reicht die Zwischenwand 10 von der Aufnahmebox 8 für den Kanülenkopf 9a bis zur Stirnwand 6 des ersten Teiles 1 der Schutzhülle. Die Seitenwände 3a, 3b, die hintere Stirnwand 6 und die Deckwand 7 umschließen als Spritzschutzwandung einen über der Zwischenwand 10 gelegenen, zur Aufnahmebox 8 hin offenen und im übrigen vollständig abgeschlossenen Einführraum für den vorderen, freien Teil der Nadel 9b der zu entsorgenden gebrauchten Kanüle 9.

Der untere Schutzhüllenteil 2 umschließt eine sich über die Länge der Schutzhülle erstreckende Aufnahmekammer 12 für eine ungebrauchte sterile Kanüle 9'. Die Kammer 12 ist bis auf eine auf der Seite der Stirnwand 5 gelegene stirnseitige Öffnung 13 vollständig abgeschlossen. Durch diese Öffnung 13 hindurch ist die ungebrauchte Kanüle 9' in die ausschließlich ihrer Unterbringung dienende Aufnahmekammer 12 über ihre Länge einführbar. Die Öffnung 13 hat eine solche lichte Weite, daß der Kanülenkopf 9a' bis zu einem Abziehbund 9c' von etwas größerem Durchmesser mit Reibschluß an der Lochwandung in diese einschiebbar ist, so daß die sterile Kanüle 9' klemmend in der Öffnung 13 gehalten ist, wenn sie in die Aufnahmekammer 12 eingebracht worden ist.

Zum Verabreichen einer Injektion oder zum Durchführen einer Blutentnahme wird die bis dahin noch sterile Schutzhülle mit der in ihr befindlichen sterilen Kanüle 9' aus einer üblichen sterilen Verpackung entnommen, dann, wie in Fig. 1 angedeutet, eine Spritze mit ihrem Hals 14 in Richtung des Pfeiles 15 (Fig. 1) in den Kanülenkopf 9a' eingesteckt und dann die an der Spritze befestigte Kanüle 9' aus der Aufnahmekammer 12 herausgezogen. Anschließend wird die Schutzhülle zunächst beiseite gelegt. Nach Beendigung der Arbeit mit der Spritze wird die Schutzhülle wieder in die Hand genommen, und zwar so, daß sie zwischen den Fingern in der Nähe der Aufnahmebox 8 von beiden Seiten her gefaßt wird, wobei die Hand mit den Fingern unterhalb der Oberkante der Schutzhülle bleibt. Sodann wird der vordere Endabschnitt der Nadel 9b in den Einführraum 11a unterhalb der Deckwand 7 eingeführt, was mühe- und gefahrlos durchführbar ist, weil zum einen die stirnseitige Eintrittsöffnung zum Einführraum 11a eine erhebliche lichte Weite hat und zum anderen die Finger der entsorgenden Person durch die über die Zwischenwand 10 hochgezogenen Seitenwände 3a und 3b der Schutzhülle jederzeit vor der Nadel, insbesondere der Nadelspitze, geschützt sind. Der Abstand zwischen der der Stirnwand 5 zugewendeten Stirnkante der Deckwand 7 und dieser Stirnwand 5 ist kleiner als die Länge der Kanüle 9, so daß das vordere Ende der Nadel 9b in den Einführraum 11a eingebracht worden sein muß, bevor die Kanüle 9 mit ihrem Kopf 9a in die Aufnahmebox 8 abgesenkt werden kann. Dieses Absenken geschieht gleichzeitig mit einem Durchdrücken der Nadel 9b von oben her durch die elastisch nachgiebige und sich dabei schlitzartig öffnende Zwischenwand 10 hindurch in den unter dieser befindlichen Aufnahmeraum 11b oberhalb des Bodens 4. Sobald die Nadel 9b durch die Zwischenwand 10 hindurchgedrückt worden ist, federn die diese bildenden Zungen 10a wieder hoch, so daß die gebrauchte Kanüle 9 nunmehr unter ihnen sicher festgehalten ist.

Es kommt gelegentlich vor, daß an der Nadelspitze nach dem Verabreichen der Injektion oder auch nach einer Blutentnahme ein Flüssigkeitstropfen hängt und mit der Nadel 9b in den Einführraum 11a hineingebracht wird. Beim Durchfedern der Nadel 9b und der Zungen 10a nach dem Hindurchdrücken durch die Zwischenwand 10 kann dieser Flüssigkeitstropfen, der möglicherweise hoch infektiös ist, versprüht werden, doch bedeutet dies keine Gefahr für die Umgebung, weil die versprühten Tröpfchen von den den Einführraum 11a und den Aufnahmeraum 11b seitwärts, nach oben und stirnseitig begrenzenden Wänden 3a, 3b, 6 und 7 abgefangen und in diesen Räumen zurückgehalten werden. Es besteht auch keine Gefahr, daß sie durch die offene Stirnseite des Einführraumes 11a nach außen gelangen, weil das Nadelende bei entsprechender Bemessung der Länge der Deckwand 7 weit genug in den Einführraum 11a hineingeschoben werden muß, bevor die Kanüle 9 in die Schutzhülle abgesenkt werden kann.

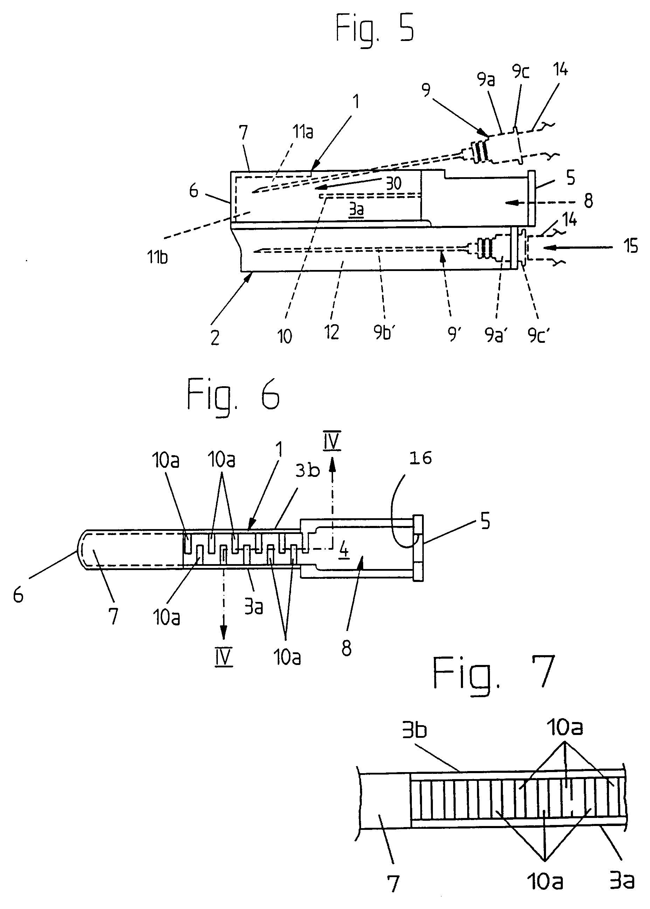

Wie aus Fig. 2 und 3 ersichtlich, ist die vordere Stirnwand 5 mit einem durchgehenden Schlitz 16 versehen, dessen Breite kleiner ist als der Abstand zwischen den Seitenwänden 3a und 3b im Bereich der Box 8. Die Breite des Schlitzes 16 ist auch kleiner als der Durchmesser eines Abziehbundes 9c am Kanülenkopf 9a aber etwas größer als der Hals 14 der Spritze, so daß die in die Schutzhülle entsorgte Kanüle 9 mittels des aus der Stirnwand 5 und dem Schlitz 16 bestehenden Abstreifers zusammen mit der Schutzhülle von dem Spritzenhals 14 abziehbar ist.

Die Ausführung gemäß Fig. 5 und 6 unterscheidet sich von der vorstehend beschriebenen Ausführungsform gemäß Fig. 1 bis 4 dadurch, daß sich die Zwischenwand 10 von der Aufnahmebox 8 für den Kanülenkopf 9a aus nur bis zur Eintrittsöffnung in den Einführraum 11a, also bis etwa zur Vorderkante der Deckwand 7, erstreckt. Diese Länge reicht aus, um die durch die Zwischenwand 10 hindurchgedrückte gebrauchte Kanüle sicher in dem Aufnahmeraum 11a festzuhalten. Andererseits federt die Nadel 9b wegen der geringeren Widerstandskraft beim Durchdrücken auch weniger durch, und es sind im Bereich des vorderen Nadelendes auch keine zurückschnellenden Zungen vorhanden, so daß insgesamt der Spritzeffekt für einen eventuell an der Nadelspitze hängenden Tropfen verringert ist.

In den Draufsichten gemäß Fig. 2 und Fig. 6 sind die Zungen 10a zur besseren Kenntlichmachung ihres Zusammenwirkens und ihrer wechselweisen Anordnung an gegenüberliegenden Seitenwänden mit einem gegenseitigen seitlichen Abstand und einem Abstand ihres freien Endes von der jeweils benachbarten Seitenwand dargestellt. Vorzugsweise grenzen sie jedoch tatsächlich gemäß Fig.1 eng aneinander an und reichen mit ihrem freien Ende auch jeweils bis an die gegeenüberliegende Seitenwand 3a bzw. 3b heran. Dies gilt sowohl für die Ausführung gemäß Fig. 1 bis 4 als auch für die Ausführung gemäß Fig. 5 und 6.

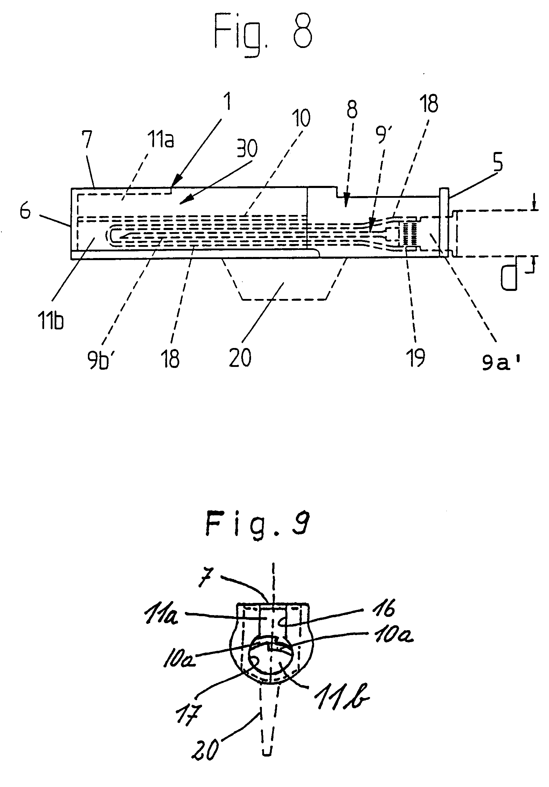

Die Ausführung gemäß Fig. 8 und 9 unterscheidet sich von den vorstehend beschriebenen Ausführungsformen dadurch, daß für die sterile Kanüle 9' keine gesonderte Aufnahmekammer vorgesehen ist. Bei ihr wird der Aufnahmeraum 11b für die zu entsorgende Kanüle 9 gleichzeitig als Aufnahmekammer für die sterile Kanüle 9' verwendet. Zu diesem Zweck ist, wie aus Fig. 9 ersichtlich, der Schlitz 16 in der Stirnwand 5 an seinem unteren Ende zu einer kreisrunden Öffnung 17 erweitert, die einen der lichten Weite der Öffnung 13 bei den vorstehend beschriebenen Ausführungsformen gemäß Fig. 1 - 6 entsprechenden Durchmesser aufweist, so daß in ihr die sterile Nadel 9' mit ihrem Kopf 9a' ebenso festgehalten werden kann, wie in der Öffnung 13 bei den anderen Ausführungen. Die sterile Kanüle 9' kann mit einer von der Nadelspitze bis zum Kanülenkopf reichenden, sterilen Schutzhülse 18 versehen sein, die auf einen nadelseitigen, zylindrischen Abschnitt 19 am Kanülenkopf 9a' aufsteckbar ist, dessen Durchmesser kleiner ist als der Durchmesser des Abschnitts, mit dem der Kanülenkopf 9a' in der Öffnung 17 festlegbar ist.

Eine solche Schutzhülse könnte auch für die sterile Kanüle 9' bei den Ausführungsformen gemäß Fig. 1 bis 6 vorgesehen werden.

In Fig. 8 ist die Schutzhülle mit einer bis zur vorderen Stirnwand 6 reichenden Zwischenwand 10 dargestellt. Es ist offensichtlich, daß bei der Ausführungsform gemäß Fig. 8 und 9 auch die Alternative gemäß Fig. 6 mit der kürzeren Zwischenwand 10 anwendbar ist.

Die gesonderte Aufnahmekammer für die sterile Kanüle 9' bei den Ausführungen gemäß Fig. 1 bis 6 erleichtert das Halten der Schutzhülle beim Entsorgen der gebrauchten Kanüle 9. Bei der Ausführungsform gemäß Fig. 8 und 9 kann ein sich vom Boden 4 abwärts erstreckender, lappenartiger und ggf. auch biegsamer Ansatz 20 zum Erleichtern des Haltens der Schutzhülle vorgesehen sein.

Am Lappen 20 und/oder an den Seitenwänden der Hülle im Abstand vom Einführraum 11a können zur weiteren Erleichterung des Haltens der Schutzhülle außen auch besondere Griffrippen ausgebildet sein.

Bei den bisher besprochenen Ausführungsformen erstreckt sich die der Verschlußwand gegenüberliegende obere Wand der Spritzschutzwandung parallel zu der Verschlußwand, so daß diese obere Wand über die Länge des Spritzschutzraumes von der Verschlußwandung überall den gleichen relativ großen Abstand hat wie bei der Eintrittsöffnung, der dort zum leichten Einführen der Nadel vorteilhaft ist. Außerdem setzen die Zungen 10a dem Durchdrücken der Nadel einen erheblichen Widerstand entgegen, weil die Nadel nicht an ihrem Ende sondern in ihrer Mitte angreift, so daß sie bei einer gegebenen Dicke, die herstellungstechnisch nicht beliebig klein gewählt werden kann, und bei einem gegebenen Elastizitätsmodul des Hüllenmaterials, das genügend hart und unelastisch sein muß, um ein Durchstechen der Nadel auszuschließen, sehr steif sind. Insbesondere bei den häufig verwendeten dünneren Nadeln besteht bei den Schutzhüllen gemäß Fig 1-9 somit eine gewisse Gefahr, daß mit ihnen nicht die zum Durchdrücken durch die Verschlußwand nötige Kraft ausübbar ist, weil sie sich schon bei einer relativ geringen Krafteinwirkung verbiegen. Hinzu kommt, daß auch eine gewisse Gefahr besteht, daß die gebrauchte Kanüle, selbst wenn mit ihr die nötige Kraft aufgebracht werden kann, aus Unachtsamkeit auf der Verschlußwand verbleibt, weil sie sich wegen der überall großen Bewegungsfreiheit von dieser weg beim Niederschwenken des Kanülenkopfes zunächst nur auf die Verschlußwand legen kann, wenn nicht gleichzeitig ein ausreichender Druck auf sie ausgeübt wird. Bei dem stets freien mittigen Schlitz, der bei den bekannten Schutzhüllen vorgesehen ist, liegen die vorgenannten Probleme nicht vor, doch besteht durch einen solchen Schlitz von der Kanüle im Aufnahmeraum freier Zugang zurück in die Umgebung, so daß infektiöse Flüssigkeit weitgehend ungehindert wieder nach außen tropfen kann und die Schutzhülle damit ihre Aufgabe, wenn überhaupt, so nur sehr unvollkommen erfüllt.

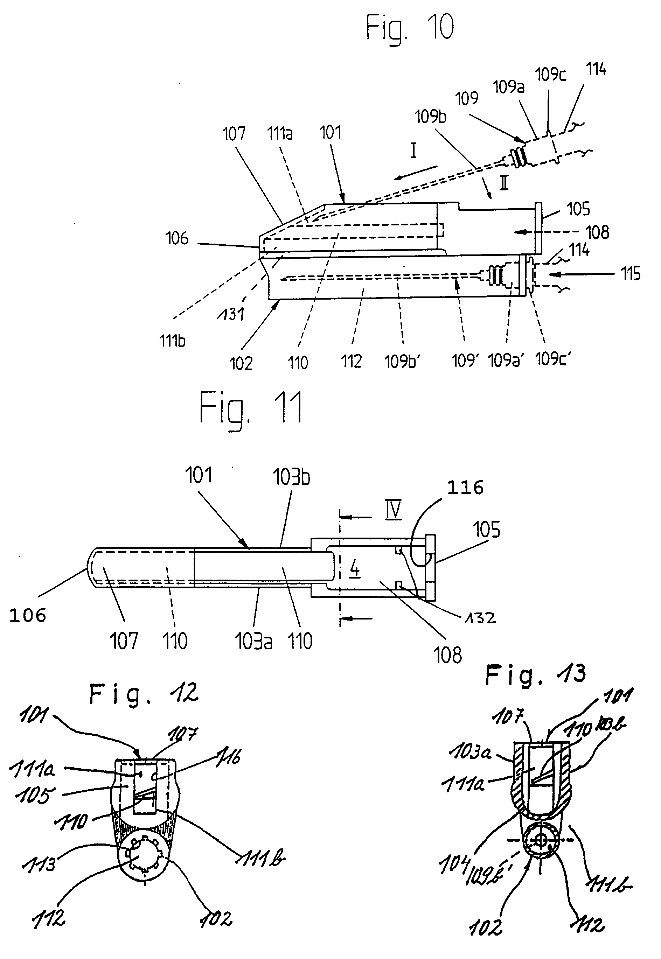

Die Ausführungen gemäß Fig. 10 bis 16 beseitigen auch die vorstehend genannten gewissen Gefahren der Ausfürungen gemäß Fig. 1 bis 9 gewährleisten insbesondere auch unter kritischen Bedingungen hinsichtlich der mit einer Kanüle ausübbaren Kraft ein sicheres Einbringen in den Aufnahmeraum nach dem Gebrauch der Kanüle. Die Ausführungen gemäß Fig. 1 bis 9 vermögen jedoch ebenfalls in vielen Anwendungsfällen sehr befriedigende Ergebnisse zu liefern.

Die Schutzhülle gemäß Fig. 10 - 13 weist einen oberen Teil 101 und einen unteren Teil 102 auf, die ebenfalls aus Kunststoff bestehen und in einem Stück z.B im Spritzgußverfahren hergestellt sein können. Der obere Teil 101 hat Seitenwände 103a, 103b, einen Boden 131, eine vordere Stirnwand 105, eine hintere Stirnwand 106 und eine Deckwand 107, die sich, ausgehend von der hinteren Stirnwand 106 über einen Teil der Hüllenlänge zwischen den Stirnwänden 105 und 106 erstreckt. In einem an die vordere Stirnwand 105 angrenzenden Bereich der Schutzhüllenlänge bilden die Seitenwände 103a, 103b mit dem Boden 131 eine für den Betrachter der Fig. 10 nach oben offene Aufnahmebox 108 für den Kopf 109a einer Kanüle 109, deren Nadel mit 109b bezeichnet ist.

Im Abstand von der Deckwand 107 und vom Boden 131 erstreckt sich zwischen der Aufnahmebox 108 und der hinteren Stirnwand 106 eine Verschlußwand 110, die sich vorzugsweise parallel zum Boden 131 erstreckt und sich, von der Seitenwand 103b ausgehend, etwas schräg abwärts zum Boden geneigt bis an die andere Seitenwand 103a heran erstreckt, ohne mir dieser verbunden zu sein, so daß sie unter Bildung oder Vergrößerung eines Spaltes zwischen sich und der Wand 103a für den Durchgang der Kanülennadel 109b elastisch zum Boden 4 hin abbiegbar ist. Die Verschlußwand 110 stellt damit ebenso wie die Verschlußwand 10 bei den vorher besprochenen Ausführungsformen einen elastisch nachgiebigen Verschluß in einem seitlichen Durchgang zu einem Aufnahmeraum 111b für die zu entsorgende gebrauchte Kanüle 109 dar. wobei sie allerdings im Unterschied zu der Verschlußwand 10 die Kanülennadel 109b vollständig und ohne den Spalten zwischen den Lappen 10a entsprechende Durchlässe für Staub und. dgl. geschlossen abdeckt. Die Kanülennadel befindet sich in der Mitte unter der Verschlußwand 110 und wird durch am Kanülenkopf angreifende Zentriernocken (132) zusätzlich in dieser mittigen Lage zentriert, wenn sie in den Aufnahmeraum 111b entsorgt worden ist, was im wesentlichen in der gleichen Weise unter Verwendung des Einführungsraumes 111a geschieht, wie bz.B. bei der Ausführung gemäß Fig. 1.

Zur Erleichterung des Heranführens der Kanülennadelspitze an den seitlichen Spalt zwischen der Wand 103a und der Verschlußwand 110 und um sicherzustellen, daß die Nadel 109b beim Absenken des Kanülenkopfes nach dem Einführen der Nadelspitze in den Einführraum 111a nicht über die Verschlußwand 110 gelangt und dort liegen bleibt, bildet die Deckwand der Haube über dem vorderen Ende des seitlichen Durchtrittsschlitzes für die Kanülennadel 109b eine Schräge 107, die mit ihrem vorderen, für den Betrachter der Fig. 10 und 11 rechten Ende bis unter die tiefste stelle der Verschlußwand 110 reicht und so die Nadelspitze beim Einführen in den Einführraum 111a zwangsweise in den Aufnahmeraum 111b leitet, wo diese dann beim Abwärtsschwenken des Kanülenkopfes 109a gehalten wird, so daß danach sich auch die ganze Nadel in den Aufnahmeraum 111b gelangt.

An ihrem der Aufnahmebox 108 für den Kanülenkopf 109a zugewendeten Ende bildet die Verschlußwand 110 einen ein Stück frei in die Box 108 vorragenden Lappen 110a, der gewährleistet, daß die Kanülennadel 109b bis zu dem Kanülenkopf 109a hin vollständig von der Verschlußwand abgedeckt wird, so daß keine Teile von ihr frei zugänglich liegen bleiben, wenn sie im entsorgten oder auch im sterilen Zustand sich in dem Aufnahmeraum 111b befindet.

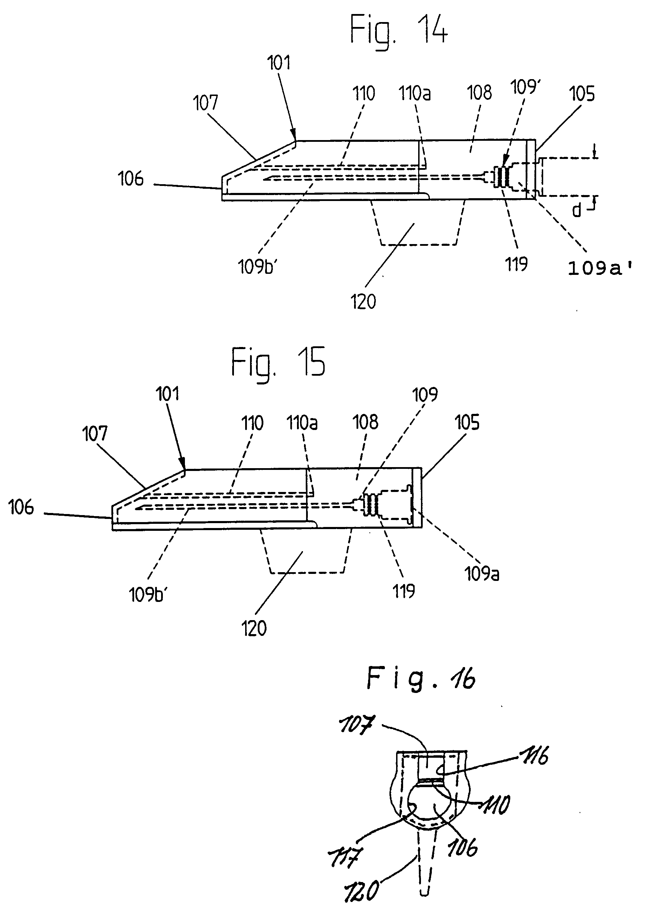

Wie bei den vorstehend besprochenen Ausführungsformen, kann auch bei der Ausführung mit von einer Seitenwand zur anderen durchgehend geschlossener Verschlußwand 110 ein gesoderter Aufnahmeraum für die sterile Kanüle 109' vorgesehen sein oder der der Entsorgung dienende Aufnahmeraum 111b auch für die Aufnahme der sterilen Kanüle verwendet werden. Ebenso können Griffrippen und Grifflappen voorgesehen sein. Entsprechende Teile sind bei der Ausführung gemäß Fig. 10 bis 13 und auch bei der nachstehend noch erörterten Ausführung gemäß Fig. 14 und 15 mit entsprechenden, um den Zahlenwert 100 erhöhten Bezugszahlen bezeichnet.

Bei der Ausführung gemäß Fig. 14 und 15, die eine Schutzhülle ohn gesonderten Afnahmeraum für die sterile Kanüle zeigt, ist die Verschlußwand 110 senkrecht zu den Seitenwänden 103a und 103b ausgerichtet, erstreckt sich also nicht unter einer gewissen Neigung von der Befestigungsstelle an der einen Seitenwand zur anderen. Eine solche Ausführung kann mit kleinerer Höhe gabaut werden als diejenige gemäß Fig. 10 bis 13, hat aber keine ganz so gute Führungswirkung auf die Nadelspitze beim Zuführen vorne in den Schlitz zwischen Seitenwand 103a und Verschlußwand 110.

Hinsichtlich des Kanülenabstreifers entsprechen die Ausführungsformen gemäß Fig. 10 bis 16 denjenigen gemäß Fig. 1 bis 9.

Bei der Ausführungen gemäß Fig. 10 bis 16 wird also die Spitze der Nadel der gebrauchten Kanüle beim Einführen in den Spritzschutzraum durch die Schräge der Spritzschutzwandung zwangsweise an die Verschlußwand herangeführt, so daß der Benutzer mit ihr leicht den aus Gründen der Aufrechterhaltung einer tropfsicheren Aufbewahrung der gebrauchten Kanüle im Aufnahmeraum seitlich außermittig zwischen der Abdeckwand und der einen Seitenwand der Schutzhülle liegenden Durchgangsschlitz für die Nadel finden kann. In diesem Zusammenhang ist es besonders günstig, wenn die Schräge zum stirnseitigen Ende der Schutzhülle hin bis unter die Höhe der Abdeckwand reicht und wenn die Verschlußwand in seitlicher Richtung von der Hüllenseitenwand, von der sie ausgeht, zum Boden des Aufnahmeraums hin geneigt ist, weil dann die Nadelspitze zwangsweise direkt in den Durchgangsschlitz geführt wird. Die seitliche Anordnung des Durchgangsschlitzes stellt für die leichte Einführbarkeit der Nadel in den Aufnahmeraum kein Hindernis dar, weil jede Nadel ein gewisses Federungsvermögen hat und auch die Abdeckwand bei der Schlitzkante aufgrund ihres dort relativ großen Abstandes von der Hüllenseitenwand, an der sie befestigt ist, federnd zurücktreten kann, so daß sich der mittig bleibende Kanülenkopf, der zur mittigen Zentrierung der Kanülennadel unter der Abdeckwand beiträgt, beim Durchführen der Nadel durch den Schlitz nicht störend auswirkt. Die Kanüle ist im Aufnahmeraum von der Abdeckwand nach außen hin so zuverlässig abgedeckt, daß die Kanüle auch vor dem Gebrauch im sterilen Zustand darin untergebracht werden kann. Da die Kanüle zur leichten Entnehmbarkeit mit der Spritze in diesem Falle mit außen liegendem Kanülenkopfbund in der Schutzhülle angeordnet ist und damit nicht so weit wie im entsorgten Zustand in die Schutzhülle hineinragt, ist es vorteilhaft, die Abdeckwand als federnden Lappen ein Stück in die Aufnahmebox für den Kanülenkopf hineinragen zu lassen. Dieser Lappen deckt einerseits die sterile Nadel bis zum Kanülenkopf ab und vermag andererseits federnd unter dem nadelseitigen Ende des Kanülenkopfes nachzugeben und dieses nach unten vorbeizulassen, wenn die Kanüle nach dem Gebrauch etwas weiter als vorher im sterilen Zustand in die Schutzhülle eingeschoben in diese entsorgt wird.

Die Zentrierlappen in der Aufnahmebox für den Kanülenkopf dienen der zusätzlichen mittigen Zentrierung der unter der Verschlußwand im Aufnahmeraum befindlichen Kanülennadel.

## Patentansprüche

1. Zur Entsorgung einer gebrauchten Einmalkanüle dienende Schutzhülle, deren Wandung einen langgestreckten Aufnahmeraum (11b;111b) für die gebrauchte Kanüle (9;109) umschließt, der mindestens so lang ist wie die Kanüle (9;109) und in den diese von der Seite her durch einen sich über dessen Länge erstreckenden seitlichen Durchgang hindurch einführbar ist, der mindestens über einen Teil der Länge der Kanülennadel (9b;109b) mittels einer Verschlußwand (10;110) elastisch nachgiebig verschlossen ist, wobei an der einen Stirnseite des Aufnahmeraums (11b;111b) ein fluchtrecht mit dem Durchgang zur Seite offener Kanülenabstreifer (5,16;105,116) zum Hintergreifen der noch auf der Injektionsspritze (14;114) steckenden, in den Aufnahmeraum (11b;111b) eingeführten Kanüle (9;109) angebracht ist, **gekennzeichnet** durch eine Spritzschutzwandung (3a,3b,6,7;-103a,103b,106,107), die sich über den von dem Kanülenabstreifer (5,16;105,116) entfernten Endbereich des Durchgangs erstreckt und außen über diesem einen zum Kanülenabstreifer (5,16;105,116) hin offenen und im übrigen geschlossenen Einführraum (11a;111a) umgrenzt, in den die Kannülennadel (9b;109b) über einen an ihre Spitze anschließenden Bereich vor dem Durchdrücken der Kanüle (9;109) durch den Durchgang einführbar ist und dessen Eintrittsöffnung von dem Kanülenabstreifer (5,16;105,116) einen Abstand hat, der kleiner ist als die Kanülenlänge.

2. Schutzhülle nach Anspruch 1, **dadurch gekennzeichnet**, daß der Aufnahmeraum (11b;111b) auch zum Aufbewahren einer unbenutzten Kanüle (9';109') mit sterilem Überzug (18;118) eingerichtet ist, die von der Stirnseite her durch den Kanülenabstreifer (5,16,17;105,116,117) hindurch in den Aufnahmeraum (11b;111b) einführbar ist.

3. Schutzhülle nach Anspruch 2, **dadurch gekennzeichnet**, daß der Kanülenabstreifer (5,16,17;105,116,117) so gestaltet ist, daß der Kanülenkopf (9a;109a) im eingeführten Zustand nadelspitzenseitig vor einem Wulst (9c;109c) gegen ihn verklemmbar ist.

4. Schutzhülle nach Anspruch 1, **gekennzeichnet** durch eine sich parallel zu dem Aufnahmeraum (11b;111b) erstreckende, von diesem gesonderte und von einer Stirnseite her zugängliche Aufnahmekammer (12:112) für eine sterile ungebrauchte Kanüle (9';109').

5. Schutzhülle nach Anspruch 4, **dadurch gekennzeichnet,** daß die Aufnahmekammer (12;112) von derjenigen Stirnseite der Schutzhülle her zugänglich ist, an der der Kanülenabstreifer (5,16;105,116) angeordnet ist.

6. Schutzhülle nach einem der vorhergehenden Ansprüche, **gekennzeichnet** durch Griffrippen, die im Abstand von dem Einführraum (11a;111a) für die Nadelspitze seitlich an der Schutzhülle ausgebildet sind.

7. Schutzhülle nach einem der vorhergehenden Ansprüche, **gekennzeichnet** durch einen sich bezüglich der Längsachse des Aufnahmeraums (11b;111b) seitwärts erstreckenden Griff-Lappen (20;120) an der Außenseite der Schutzhülle.

8. Schutzhülle nach einem der vorhergehenden Ansprüche, **gekennzeichnet** durch sich beiderseits des Durchgangs im gegenseitigen Abstand etwa parallel zueinander vom Durchgang weg außwärts erstreckende Wände (3a,3b) zum Schutz gegen Berühren der gebrauchten Kanüle (9) mit der Hand beim Handhaben der Schutzhülle.

9. Schutzhülle nach einem der vorhergehenden Ansprüche, **dadurch gekennzeichnet**, daß die Verschlußwand (110) als geschlossene Abdeckwand von der einen, mit ihr verbundenen Seitenwand (103b) der Schutzhülle bis unmittelbar vor deren andere Seitenwand (103a) reicht und daß die Spritzschutzwandung (103a,103b,106,107) eine Schräge (107) bildet, die sich von der Oberseite der Eintrittsöffnung (130) des Einführraumes (111a) abwärts zur Verschlußwand (110) hin bis zu dem vom Kanülenabstreifer (105,116) abgewendeten Ende der Schutzhülle erstreckt.

10. Schutzhülle nach Anspruch 9, **dadurch gekennzeichnet**, daß sich die Schräge (107) zum Ende der Schutzhülle hin bis unter die Höhe erstreckt, die die Verschlußwand (110) über dem Boden (131) des Aufnahmeraums (111b) einnimmt.

11. Schutzhülle nach Anspruch 9 oder 10, **dadurch gekennzeichnet**, daß die Verschlußwand (110) in Richtung senkrecht zu ihrer Längserstreckung von der mit ihr verbundenen Seitenwand (103b) abwärts zum Boden (131) des Aufnahmeraums (111b) hin geneigt ist.

12. Schutzhülle nach einem der Ansprüche 9 bis 11, **dadurch gekennzeichnet**, daß die Verschlußwand (110) ein Stück in eine Aufnahmebox (108) für den Kanülenkopf (109a) hineinragt und dort einen zu den Seiten und zur Aufnahmebox (108) hin freien elastischen Lappen (110b) bildet.

13. Schutzhülle nach einem der Ansprüche 9 bis 12, **gekennzeichnet** durch im Bereich des eingeführten Kanülenkopfes (109a) im Abstand vom Kanülenabstreifer (105,116) in der Schutzhülle angebrachte Zentriernocken (132) zum Zentrieren der in den Aufnahmeraum (111b) eingeführten Nadel (109) auf dessen Mitte.

14. Schutzhülle nach einem der Ansprüche 1 bis 8, **dadurch gekennzeichnet,** daß die Verschlußwand (10) im Bereich der eingeführten Kanülennadel (9b) aus sich gegeneinander erstrekkenden, ineinandergreifenden, seitwärts aneinander angrenzenden und an den freien Enden bis an die Seitenwände (3a,3b) der Schutzhülle heranreichenden elastischen Fingern reißverschlußähnlich gebildet ist.

## Claims

1. Protective sleeve which serves for the waste disposal of a used disposable cannula and the wall of which encloses an elongate receiving space (11b; 111b) for the used cannula (9; 109), which space is at least as long as the cannula (9; 109) and into which this is introducible from the side through a lateral passage which extends over its length and which is closed in elastically yielding manner by means of a closure wall (10; 110) over at least a part of the length of the cannula needle (9b; 109b), wherein a cannula stripper (5, 6; 105, 116), which is open to one side truly aligned with the passage, is arranged at the one end face of the receiving space (11b; 111b) for engaging behind the cannula (9; 109) still plugged onto the syringe (14; 114) and introduced into the receiving space (11b; 111b), characterised by a spray protection wall (3a, 3b, 6, 7; 103a, 103b, 106, 107), which extends over that end region of the passage, which is remote from the cannula stripper (5, 16; 105, 116), and externally over this passage bounds an introduction space (11a; 111a), which is open towards the cannula stripper (5, 16; 105, 116) and closed for the remainder and into which the cannula needle (9b; 109b) is introducible over a region, which adjoins its tip, through the passage before the pressing-through of the cannula (9; 109) and the entry opening of which has a spacing, which is smaller than the length of the cannula, from the cannula stripper (5, 16; 105, 116).

2. Protective sleeve according to claim 1, characterised thereby, that the receiving space (11b; 111b) is also arranged for the keeping of an unused cannula (9'; 109') with a sterile covering (18; 118), which cannula is introducible from the end face through the cannula stripper (5, 16, 17; 105, 116, 117) into the receiving space (11b; 111b).

3. Protective sleeve according to claim 2, characterised thereby, that the cannula stripper (5, 16, 17; 105, 116, 117) is so structured that the cannula head (9a; 109a) in the introduced state is clampable at the needle tip side against a head (9c; 109c).

4. Protective sleeve according to claim 1, characterised by a receiving chamber (12; 112), which extends parallelly to the receiving space (11b, 111b), is separate from this and accessible from one end face, for a sterile unused cannula (9'; 109').

5. Protective sleeve according to claim 4, characterised thereby, that the receiving chamber (12; 112) is accessible from that end face of the protective sleeve, at which the cannula stripper (5, 16; 105, 116) is arranged.

6. Protective sleeve according to one of the preceding claims, characterised by handle ribs which are formed laterally at the protective sleeve at a spacing from the introduction space (11a; 111a) for the needle tip.

7. Protective sleeve according to one of the preceding claims, characterised by handle lobe (20; 120), which extends laterally with respect to the longitudinal axis of the receiving space (11b; 111b), at the outward side of the protective sleeve.

8. Protective sleeve according to one of the preceding claims, characterised by wall (3a, 3b), which extend outwardly away from the passage about parallelly one to the other at a mutual spacing on both sides of the passage, for protection against touching the used cannula (9) by hand during the manipulation of the protective sleeve.

9. Protective sleeve according to one of the preceding claims, characterised thereby, that the closure wall (110) as closed cover wall reaches from the one side wall (103b), which is connected with it, of the protective sleeve to directly in front of its other side wall (103a) and that the spray protection wall (103a, 103b, 106, 107) forms a slope (107), which extends from the upper side of the entry opening (130) of the introduction space (111a) downwardly towards the closure wall (110) to that end of the protective sleeve, which is remote from the cannula stripper (105, 116).

10. Protective sleeve according to claim 9, characterised thereby, that the slope (107) extends towards the end of the protective sleeve to below the height which the closure wall (110) takes up above the base (131) of the receiving space (111b).

11. Protective sleeve according to claim 9 or 10, characterised thereby, that the closure wall (110) in the direction perpendicular to its longitudinal extent is sloping downwardly from the side wall (103b), which is connected with it, towards the base (131) of the receiving space (111b).

12. Protective sleeve according to one of the claims 9 to 11, characterised thereby, that the closure wall (110) projects somewhat into a receiving box (108) for the cannula head (109a) and there forms an elastic lobe (110b), which is free towards the sides and the receiving box (108).

13. Protective sleeve according to one of the claims 9 to 12, characterised thereby, that centring dogs (132), which are mounted in the protective sleeve at a spacing from the cannula stripper (105, 116) in the region of the introduced cannula head (109a), for the centring of the needle (109), which is introduced into the receiving space (111b), to the centre thereof.

14. Protective sleeve according to one of the claims 1 to 8, characterised thereby, that the closure wall (10) in the region of the introduced cannula needle (9b) is formed of interengaging elastic fingers, which extend one towards the other, adjoin one laterally against the other and at the free ends reach to the side walls (3a, 3b) of the protective sleeve, similarly to a zip fastener.

## Revendications

1. Housse de protection, qui sert à se débarrasser d'une canule usagée à usage unique, dont la paroi entoure une zone (11b; 111b) oblongue de réception de la canule (9; 109) usagée qui est au moins aussi longue que la canule (9; 109) et dans laquelle la canule peut être introduite depuis le côté en la faisant passer par un passage latéral, lequel s'étend sur toute la longueur de la zone de réception et est fermée de manière pouvant céder élastiquement au moins sur une partie de la longueur de l'aiguille (9b; 109b) de la canule, au moyen d'une paroi (10; 110) de fermeture, un dispositif (5, 16; 105, 116) de retrait de la canule, ouvert vers le côté en étant aligné sur le passage et servant à accrocher la canule (9; 109) qui est encore enfichée sur la seringue (14, 114) d'injection et qui est introduite dans la zone (11b; 111b) de réception, étant monté sur un des côtés frontaux de la zone (11b; 111b) de réception, caractérisée par une paroi (3a, 3b, 6, 7; 103a, 103b, 106, 107) qui protège des projections, qui s'étend sur la région d'extrémité du passage éloignée du dispositif (5, 16; 105, 116) de retrait de la canule et qui entoure extérieurement, au-dessus de ce passage, une zone (11a; 111a) d'introduction, qui est ouverte en direction du dispositif (5, 16; 105, 116) de retrait de la canule et, pour le reste, fermée, dans laquelle l'aiguille (9b; 109b) de la canule peut être, avant d'enfoncer la canule (9; 109) au travers du passage, introduite par l'intermédiaire d'une partie faisant suite à sa pointe et dont l'ouverture d'introduction est à une distance du dispositif (5, 16; 105, 116) de retrait de la canule qui est inférieure à la longueur de la canule.

2. Housse de protection suivant la revendication 1, caractérisée en ce que la zone (11b; 111b) de réception est aménagée aussi pour conserver une canule (9'; 109') inutilisée, qui comporte un capuchon (18, 118) stérilisé et qui peut être introduite dans la zone (11b; 111b) de réception, depuis le côté frontal, en la faisant passer au travers du dispositif (5, 16; 105, 116) de retrait de la canule.

3. Housse de protection suivant la revendication 2, caractérisée en ce que le dispositif (5, 16, 17; 105, 116, 117) de retrait de la canule est réalisé de manière que la tête (9a; 109a) de la canule, lorsqu'elle est introduite, peut être bloquée du côté de la pointe de l'aiguille sur un renflement (9c; 109c), devant ce renflement.

4. Housse de protection suivant la revendication 1, caractérisée par une chambre (12; 112) de réception d'une canule (9'; 109') stérilisée non usagée qui s'étend parallèlement à la zone (11b; 111b) de réception qui en est séparée et qui est accessible d'un côté frontal.

5. Housse de protection suivant la revendication 1, caractérisée en ce que la chambre (12; 112) de réception est accessible du côté frontal de la housse de protection sur lequel est disposé le dispositif (5, 16; 105, 116) de retrait de la canule.

6. Housse de protection suivant l'une des revendications précédentes, caractérisée par des nervures de préhension qui sont formées à distance de la zone (11a; 11a) de réception de la pointe de l'aiguille, latéralement à la housse de protection.

7. Housse de protection suivant l'une des revendications précédentes, caractérisée par une patte (20; 120) de préhension qui est disposée du côté extérieur de la housse de protection et qui s'étend vers le côté par rapport à l'axe longitudinal de la zone (11b; 111b) de réception.

8. Housse de protection suivant l'une des revendications précédentes, caractérisée par des parois (3a, 3b) qui protègent la main d'un contact avec la canule (9) usagée lors du maniement de la housse de protection et qui s'étendent de part et d'autre du passage, à peu près parallèlement à une distance mutuelle, en s'éloignant du passage.

9. Housse de protection suivant l'une des revendications précédentes, caractérisée en ce que la paroi (110) de fermeture va, en tant que paroi de recouvrement fermée de l'une (103b) des parois latérales de la housse de protection qui lui est reliée directement jusque devant l'autre (103a) paroi latérale et en ce que la paroi (103a, 103b, 106, 107) protégeant des projections, forme une partie (107) oblique, qui s'étend de la face supérieure de l'ouverture (130) d'entrée de la zone (111a) d'introduction, vers le bas en direction de la paroi (110) de fermeture, jusqu'à l'extrémité de la housse de protection qui est éloignée du dispositif (105, 116) de retrait de la canule.

10. Housse de protection suivant la revendication 9, caractérisée en ce que la partie (107) oblique s'étend jusqu'à l'extrémité de la housse de protection, au-dessous du niveau que prend la paroi (110) de fermeture audessus du fond (131) de la zone (111b) de réception.

11. Housse de protection suivant la revendication 9 ou 10, caractérisée en ce que la paroi (110) de fermeture est inclinée, dans la direction perpendiculaire à son étendue longitudinale, de la paroi (103b) latérale qui lui est reliée vers le bas en direction du fond (131) de la zone (111b) de réception.

12. Housse de protection suivant l'une des revendication 9 ou 11, caractérisée en ce que la paroi (110) de fermeture dépasse d'un tronçon dans une boîte (108) de réception de la tête (109a) de la canule et y forme une patte élastique libre vers les côtés et vers la boîte (108) de réception.

13. Housse de protection suivant l'une des revendications 9 à 12, caractérisée par un toc (132) de centrage qui est monté dans la housse de protection, dans la région de la tête (109a) de la canule introduite, à distance du dispositif (105, 116) de retrait de la canule, et qui sert à centrer l'aiguille (109) introduite dans la zone (111b) de réception au milieu de cette zone.

14. Housse de protection suivant l'une des revendications 1 à 8, caractérisée en ce que la paroi (10) de fermeture est formée, dans région de l'aiguille (9b) de la canule introduite, de manière semblable à une fermeture à glissière, de doigts élastiques, qui s'étendent en sens opposé, qui s'interpénètrent, qui sont contigus les uns aux autres sur les côtés et qui, par les extrémités libres vont jusqu'aux parois (3a, 3b) latérales de la housse de protection.
